# EUROPEAN PATENT SPECIFICATION

(11) **EP 0 875 256 B1**
(45) Date of publication and mention of the grant of the patent: **17.12.2003**
(21) Application number: 98302200.5
(22) Date of filing: 24.03.1998
(51) Int. Cl.: A61M 1/00

(54) **Irrigation and evacuation cannula**
Irrigations und Evakuierungkanüle
Canule d'irrigation et d'aspiration

(30) Priority: 26.03.1997 US 824849
(43) Date of publication of application: 04.11.1998
(73) Proprietor: TYCO HEALTHCARE GROUP LP, Norwalk, Connecticut 06856 (US)
(72) Inventor: Exline, Donald Delbert, Carrolton, Texas 75006 (US); Pierce, William Arthur, Dallas, Texas 75214 (US)
(74) Representative: Marsh, Roy David

(56) References cited:
- WO-A-93/25139
- US-A- 5 244 459

## Description

The present invention relates generally to surgical instruments. More particularly, the present invention relates to cannulae and sleeves used to irrigate and evacuate body cavities during endoscopic or minimally invasive surgery. The features of the preamble of claim 1 are known from US 5,244,459.

Endoscopic surgical procedures (sometimes referred to as "laparoscopy") are becoming increasingly accepted in the medical community for many surgical procedures. In endoscopic surgery, a small incision typically is made in the abdominal wall through which a fiber-optic viewing device ("endoscope")is inserted into the abdominal or other body cavity. Other small incisions are made through which surgical instruments are passed to dissect tissue and perform other operations. Frequently, the body cavity is insufflated with an inert gas, such as CO₂, which provides space in which to manipulate surgical instruments and to view the area in which surgical operations are to take place. Endoscopic procedures are preferable to many conventional surgical techniques because they are less invasive and therefore permit patients to recover more quickly and reduce the rate of infection.

Because the endoscopic surgical site is enclosed within the body cavity, irrigation and evacuation of the site is rendered somewhat more difficult in endoscopic procedures than in open or conventional techniques.

In both open and endoscopic surgical procedures, suction and irrigation tubes are extended to the surgical site, one to evacuate fluids and one to irrigate with fluid. Provision of two separate tubes for irrigation and evacuation, such as known from WO 93/25139, is cumbersome. Commonly owned U.S. Patent No. 4,668,215, May 26, 1987 to Allgood, discloses a valving arrangement whereby irrigation and evacuation are accomplished with a single line or tube passed through an endoscopic cannula or sleeve. Similarly, commonly owned U.S. Pat. No. 4,776,840, October 11, 1988 to Freitas et al. discloses a device for simultaneously proving irrigation and evacuation that is adapted for single handed use. Nevertheless, a tube passes from the device, through a cannula or sleeve, and into the surgical site. Irrigation and evacuation tubes have been combined into endoscopic cannulae or sleeves to combine previously separate apparatus into one. This cannula is passed through an incision and provides a tube through which suction may be applied to evacuate blood and other fluids from the surgical site and through which fluid may be provided to irrigate the surgical site.

To permit the surgical instrument to pass unobstructed through the irrigation and evacuation cannula or sleeve, complex valving arrangements are required to permit connection of evacuation and irrigation apparatus with the cannula. These arrangements commonly take the form of trumpet valves which are arranged at generally right angles to the lumen or sleeve.

An example of such an arrangement can be formed in US. Patent No. 5,244,459 from which the features of the preamble of claim 1 are know. Thus, during irrigation and evacuation, fluids and bits of tissue travel through a convoluted path between the irrigation and evacuation conduits, the valves, the cannula passage, and into and out of the body cavity. Examples of such prior art cannula can be found in commonly assigned U.S. Patent No. 5,312,373, May 17, 1994 of Freitas, and in U.S. Patent No. 4,696,305 of Vong Berg. Such prior art cannulae are difficult to clean and sterilize, and thus to reuse. Additionally, the convoluted fluid path has generally poor fluid, dynamics and makes the apparatus prone to clogging.

A need exists, therefore for surgical irrigation and evacuation cannulae having improved valving axrangements that permit fluid to flow through the cannula in a more linear fashion and that are thus more easily cleaned and sterilized and less prone to clogging.

### SUMMARY OF THE INVENTION

It is a general object of the present invention to provide an improved irrigation and evacuation cannula or sleeve for use in irrigating and evacuating a bodily cavity during surgery. This and other objects of the present invention are achieved by providing a cannula comprising a housing secured to a generally tubular sleeve member. The housing is provided with irrigation and evacuation fluid ports for connection to irrigation and evacuation conduits. An instrument port is formed in the housing and aligned with the sleeve member, wherein a surgical instrument can be inserted through the instrument port, housing, and sleeve member and into the bodily cavity. The instrument port includes seal members to seal against fluid leakage from the cannula with or without a surgical instrument passing through the housing and sleeve member. A valve member is rotatably carried by the housing to selectively obstruct one or more of the irrigation and evacuation ports. The valve is arranged such that the surgical instrument may remain in the housing and sleeve member during manipulation of the valve.

According to the preferred embodiment of the present invention, a generally circular recess is provided in the housing to receive the valve member. The irrigation, evacuation, and instrument ports are arranged about the circumference of the circular recess. The valve member and recess cooperate to define a traverse, venturi shaped chamber within the housing.

In one embodiment of the present invention, the instrument port includes both a lip seal and an outer diameter seal

According to the preferred embodiment of the present invention, the valve member is selectively removable from the housing.

According to the preferred embodiment of the present invention, a fluid path extends from the instrument port, through the housing and venturi shaped chamber and the tubular sleeve member and is generally straight to provide improved fluid flow dynamics.

According to the preferred embodiment of the present invention, a pair of seal members are disposed between the valve member and housing to seal the first and second fluid ports against fluid leakage.

According to the preferred embodiment of the present invention, a switch member may be carried by the housing to cooperate with the valve member to selectively actuate a device external to the cannula, such as an irrigation or evacuation pump, responsive to movement of the valve member.

### DESCRIPTION OF THE DRAWINGS

Figures 1-3 are partial elevation views, partially in section, of the irrigation and evacuation cannula according to the present invention.
Figure 4 is an exploded, side elevation view of the housing portion of the irrigation and evacuation cannula of Figures 1-3.
Figure 5 is an enlarged, partial elevation view of a portion of the valve member illustrated in Figure 4.
Figure 6 is a plan view of the valve member illustrated in Figures 1-5.
Figure 7 is a side elevation view of the valve member of Figure 6.
Figure 8 is a partial elevation view of a portion of the housing of the irrigation and evacuation cannula according to the preferred embodiment 6 of the present invention.

### DESCRIPTION OF THE PREFERRED EMBODIMENT

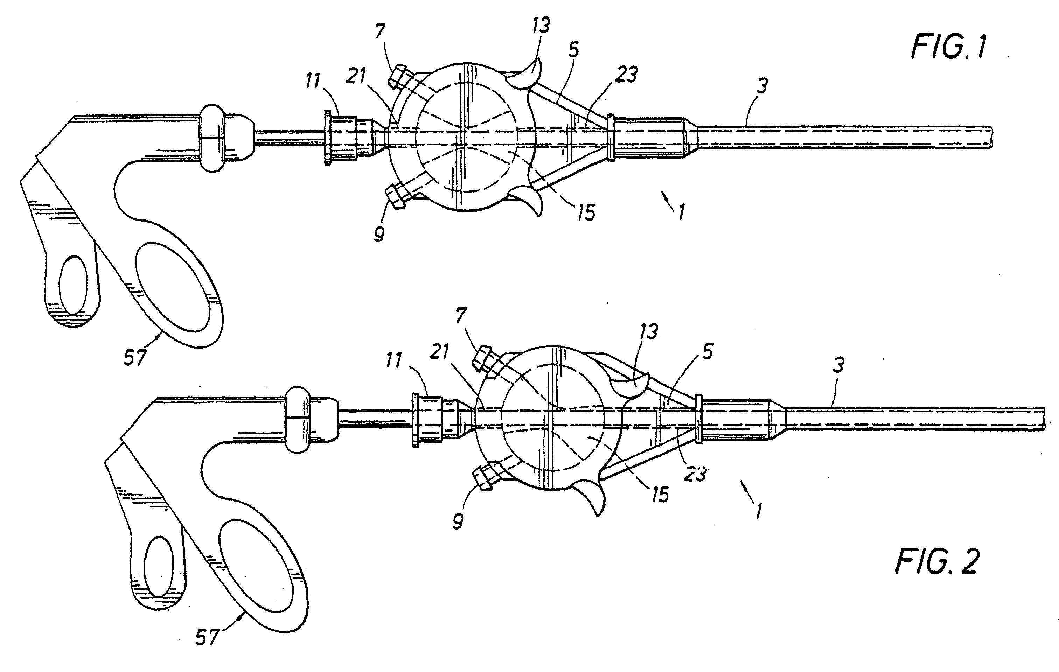

Referring now to the several views, Figures 1-3 are partial elevation views, partially in section, of the irrigation and evacuation cannula **1** according to the present invention. Irrigation and evacuation cannula **1** comprises a generally tubular sleeve member **3** having a passageway or lumen extending therethrough. The sleeve member **3** is the portion of the cannula **1** that is inserted through an incision in a body wall to irrigate and evacuate a surgical site during endoscopic surgery.

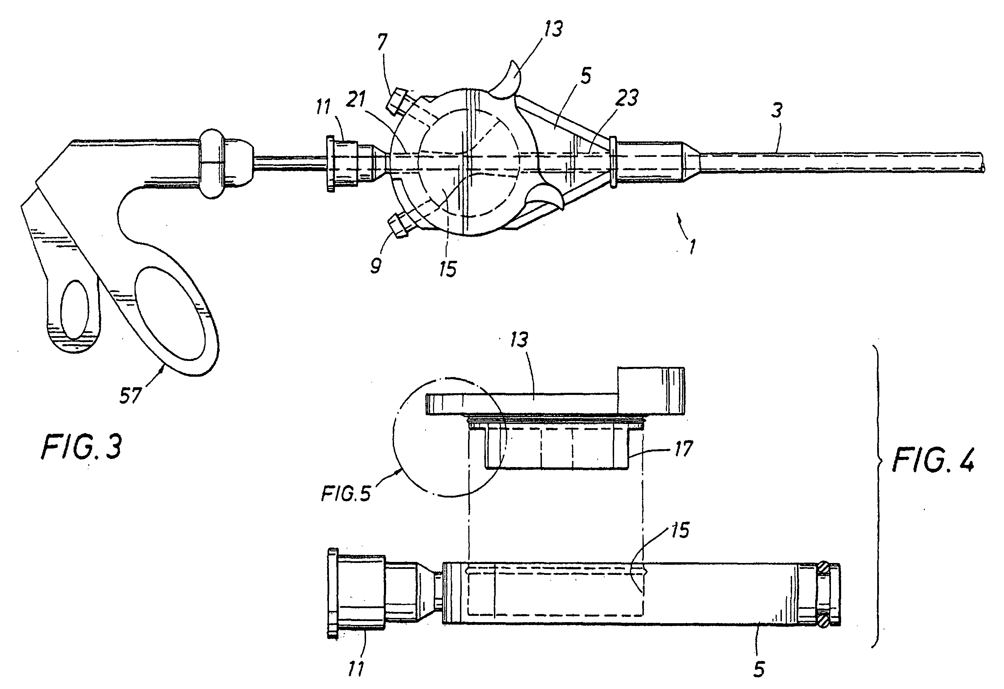

Sleeve member **3** is coupled to a housing 5 by threads, and an 0-ring (See Fig. 4) is provided to seal the joint against fluid loss. The housing **5** is provided with first and second or irrigation and evacuation ports 7 and **9** which are arranged generally opposite the sleeve member **3** and are angularly displaced less than 90° from the longitudinal axis of the cannula **1**. Irrigation and evacuation ports **7** and **9** are adapted for connection to the conduits of fluid supply (irrigation) and vacuum or suction (evacuation) apparatus (not shown). An instrument port **11** is provided in the housing **5** opposite from and aligned with the sleeve **3.** Instrument port **11** is provided with a conventional lip seal and outer diameter seal which cooperate to seal against fluid loss through port **11** whether a surgical instrument is present or not. Thus, an endoscopic surgical instrument **51** can be inserted through the instrument port **11**, then housing **5,** sleeve member **3,** and into the body cavity without loss of insufflation gas flowing through the cannula 1.

A valve member **13** is received for rotation in a circular recess **15** formed in the housing **5**. The hidden recess **15** is depicted in the phantom in Figures 1-3. The valve member **13** is formed to cooperate with the circular recess **15** in the housing **5** to define a venturi shaped chamber that is narrower or more restricted in its central region than at its periphery. The valve member **13** selectively obstructs irrigation and evacuation ports **7** and **9** which are in fluid communication with the circular recess **15** and sleeve member **3**.

The venturi shaped chamber defined by the valve member **13** and recess 15 permits selective obstruction of either or both of irrigation and evacuation ports **7** and **9** while a surgical instrument **51** extends through the housing **5** and sleeve member **3** even during manipulation of the valve **13**.

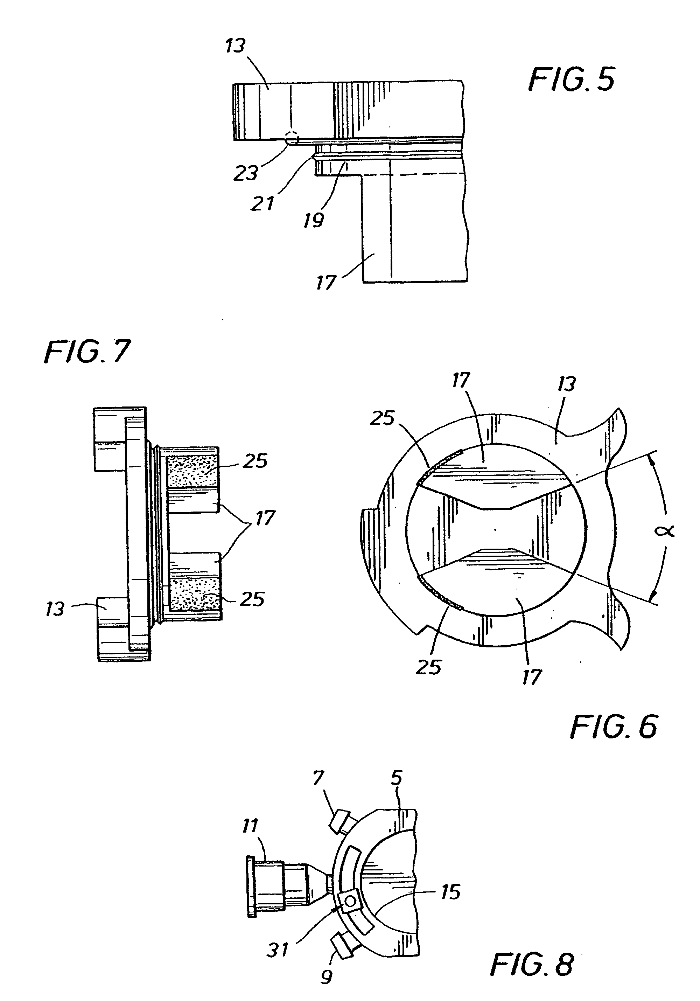

Figures 1-3 illustrate the irrigation and evacuation cannula **1** in three configurations, in each of which an endoscopic surgical instrument **51** extends through the instrument port **11** housing **5**, and sleeve member **3.** In Figure **1**, both irrigation and evacuation ports **7** and **9** are obstructed and closed by aligned portions of the valve member **13** and are sealed against fluid loss (the seals are described in greater detail with reference to Figure 6). In each configuration, the seals of the instrument port **11** prevent fluid loss around the instrument **51**.

Figure 2 illustrates the irrigation and evacuation cannula **1** in an irrigation mode in which the irrigation port **7** is open, while the evacuation port **9** is obstructed by the valve member **13**. In this configuration, irrigation fluid is introduced from an irrigation conduit (not shown) flowing through the irrigation port 7, housing 5, sleeve member **3**; and into the body cavity.

Figure 3 depicts the irrigation and evacuation cannula **1** in an evacuation configuration in which the evacuation port **9** is open and the irrigation port **7** is obstructed by the valve member **13**. In this configuration, vacuum or suction is applied from an evacuation conduit (not shown) through the evacuation port **9,** then housing **5**, sleeve member **3,** and into the body cavity.

Each of the configurations of Figures 1-3 is accomplished by rotating the valve member **13** relative to the housing 5. Because of the configuration of the housing **5** and the valve member **13,** this rotation can occur without regard to the presence (or absence) of the surgical instrument **51** in the cannula **1.** Because the ports **7** and **9** are angularly displaced from the longitudinal axis of the cannula **1**, and due to the venturi shaped chamber defined by the valve **13** and housing **5,** the flow path that fluid must follow is generally straight and avoids right angle turns, thereby promoting more efficient fluid flow and providing fewer places for debris to be caught.

Figures 4 and 5 are enlarged elevation views depicting the cooperation between the valve member **13** and circular recess **15** in the housing **5.** As shown, the valve member **13** includes a pair of projecting portions **17** that together are limited to a circular profile around the edge. Preferably, the two portions **17** cooperate to define a circle about 450m m in diameter. The circular perimeter is interrupted at the outside circular face by the ends of a transverse passage shown in Fig. 6 to permit flow. Thus, the valve member **13** is assembled into and rotatable within the circular recess **15** of the housing **5.** Venturi shaped chamber is defined between the portions **17** when enclosed within a circular recess **15** of the housing **5.**

As illustrated in Figure **5,** a circular shoulder **19,** which includes an annular radial projection **21,** is provided on the valve member **13**. THe shoulder **19** and radial projection **21** cooperate with corresponding recesses formed in the circular recess **15** of housing **5** to provide a "snap-together" assembly that is easily assembled and disassembled. An 0-ring **23** is provided in the valve member 13 to seal the joint between the valve member **13** and the circular recess **15** against fluid loss.

Figures 6 and 7 are plan and elevation views of the valve member 13 according to the present invention. Each upstanding projection **17** is tapered as illustrated to define an included angle of about 60° at the inlet and outlet ends of the venturi shaped chamber. As shown in Figure 6, two seal members **25** are formed on the circular periphery of the portions **17.** The seal members **25** preferably are formed of Santoprenetm™, a polypropylene elastomer sold by Advanced Elastomer Systems of Akron, Ohio, which is of adequate thickness, preferably 1-2mm, to be compressed when the valve member **14** is assembled into the circular recess **15** of the housing **5**. As shown in Figures 1-3, seal members **25** are locate to seal against the irrigation and evacuation ports **7** and **9** when they are obstructed by the portions **17** of the valve member **13**. The exterior of the valve member **13** is provided with a pair of lugs or ears to enable hand gripping for a mechanical advantage to operate the valve member **13** rotating in the housing **5**.

Figure **8** is a partial elevation view of the housing **5** of the irrigation and evacuation cannula 1 disclosing an additional embodiment of the present invention. In Figure 8, the housing 5 is illustrated without the valve member **13** rotatably received within the circular recess **15**. According to this embodiment of the present invention, a recesed microswitch **31** is embedded in the housing **5** beneath the flange (shown in Figures 1-3 and 6) carried by the valve member. Upon rotation of the valve member **13,** the flange toggles the microswitch **31** to actuate a suction or irrigation apparatus. Thus, first the suction wand later the irrigation apparatus is automatically actuated depending upon the position of the valve member **13** relative to the housing **5**.

The irrigation and evacuation cannula **1** is constructed of conventional materials for surgical instruments. The housing **5,** the sleeve member **3** and valve member **13** are formed of transparent or translucent polycarbonate to permit viewing of the surgical instrument **51** and fluid flow through cannula **11** in operation. All elastomeric seals are formed of conventional materials and the seals **25** are formed of the previously mentioned Santoprene™.

The irrigation and evacuation cannula according to the present invention provides a number of advantages. A principal advantage of the present invention is that it provides a cannula that is of simple design with relatively few moving parts. Another advantage is that fluid flows through the cannula in a relatively straight or linear fashion, avoiding the tortured flow paths provided in prior art cannulae. This renders the cannula according to the present invention more efficient in operation, more easily cleaned, and less prone to clogging.

The invention has been described with reference to a preferred embodiment thereof. It is thus not limited, but is susceptible to variation and modification without departing from the scope of the invention.

The invention is useful for a method of conducting endoscopic surgery, which comprises the steps of
(a) directing an elongate sleeve into a surgery site;
(b) connecting selectively but not simultaneously an irrigation flow and vacuum flow through the sleeve so that the surgery site is washed and evacuated controllably under control of the surgeon;
(c) inserting an elongate surgical instrument through the elongate sleeve to the surgical site; and
(d) while the surgical instrument is extended to the surgical site, controllably connecting the irrigation and vacuum flow to enable use of the surgical instrument.

## Claims

1. An irrigation and evacuation cannula comprising:
a housing (5) providing a chamber;
a generally tubular sleeve member (3) secured to the housing and in fluid communication with the chamber;
an instrument port (11) in the housing in fluid communication with the chamber, located generally opposite to and generally aligned with the sleeve member; and
first and second fluid ports (7, 9) in the housing in fluid communication with the chamber; **characterised by**
a valve member (13) rotatable within the housing for selective obstruction of one or both of the first and second fluid ports, while continuously permitting a surgical instrument to be inserted through the instrument port, the housing and valve member, and the sleeve member.

2. A cannula according to claim 1 wherein the valve member comprises a venturi shaped through passage for permitting the surgical instrument to pass from the instrument port to the sleeve member.

3. A cannula according to claim 2 wherein said passage is movable into fluid communication with the first and second ports to provide selective fluid coupling between said fluid port and the sleeve member.

4. A cannula according to any preceding claim further comprising a switch (31) member actuated by movement of the valve member.

5. A cannula according to any preceding claim wherein at least one valve seal member (25) is disposed between the valve member and the housing to seal said fluid ports against fluid loss.

6. A cannula according to any preceding claim wherein the valve member is selectively removable from the housing.

7. A cannula according to any preceding claim wherein said instrument port comprises a seal member.

8. A cannula according to claim 7 wherein the seal member of the instrument port includes a lip seal and an outer diameter seal.

9. A cannula according to any preceding claim wherein the first and second ports are directed into the housing at an acute angle to the instrument port so as to be approximately in line with the sleeve member.

10. A cannula according to any preceding claim wherein the valve member and the housing have matching circular cross-sections, and the valve member is rotatably accommodated within the chamber.

## Patentansprüche

1. Spül- und Abführkanüle, umfassend:
ein eine Kammer vorsehendes Gehäuse (5);
ein im Allgemeinen rohrförmiges Hülsenelement (3), das an dem Gehäuse angebracht ist und in fluider Verbindung mit der Kammer steht;
eine in fluider Verbindung mit der Kammer stehende Instrumentenöffnung (11) in dem Gehäuse, die im Allgemeinen gegenüberliegend dem Hülsenelement angeordnet und im Allgemeinen mit diesem ausgerichtet ist; und
eine in fluider Verbindung mit der Kammer stehende erste und zweite Fluidöffnung (7, 9) in dem Gehäuse, **gekennzeichnet durch**
ein Ventilelement (13), das drehbar innerhalb des Gehäuses zum gezielten Verschließen der ersten und/oder der zweiten Fluidöffnung ist, während weiterhin das Einführen eines chirurgischen Instruments **durch** die Instrumentenöffnung, das Gehäuse, das Ventilelement und das Hülsenelement möglich ist.

2. Kanüle nach Anspruch 1, wobei das Ventilelement eine venturi-förmige Durchführung zum Durchführen des chirurgischen Instruments von der Instrumentenöffnung zu dem Hülsenelement aufweist.

3. Kanüle nach Anspruch 2, wobei die Durchführung in eine fluide Verbindung mit der ersten und der zweiten Öffnung bewegbar ist, um einen gezielten Fluidanschluss zwischen der Fluidöffnung und dem Hülsenelement vorzusehen.

4. Kanüle nach einem der vorhergehenden Ansprüche, des weiteren umfassend ein Schaltelement (31), das durch die Bewegung des Ventilelements betätigt wird.

5. Kanüle nach einem der vorhergehenden Ansprüche, wobei zumindest ein Ventilabdichtelement (25) zwischen dem Ventilelement und dem Gehäuse angeordnet ist, um die Fluidöffnungen gegen einen Fluidverlust abzudichten.

6. Kanüle nach einem der vorhergehenden Ansprüche, wobei das Ventilelement gezielt von dem Gehäuse abnehmbar ist.

7. Kanüle nach einem der vorhergehenden Ansprüche, wobei die Instrumentenöffnung ein Abdichtelement aufweist.

8. Kanüle nach Anspruch 7, wobei das Abdichtelement der Instrumentenöffnung eine Lippendichtung und eine Außendurchmesserabdichtung umfasst.

9. Kanüle nach einem der vorhergehenden Ansprüche, wobei die erste und die zweite Öffnung in das Gehäuse unter einem spitzen Winkel zu der Instrumentenöffnung gerichtet sind, und so ungefähr auf gleicher Linie mit dem Hülsenelement liegen.

10. Kanüle nach einem der vorhergehenden Ansprüche, wobei das Ventilelement und das Gehäuse einander entsprechende kreisförmige Querschnitte besitzen, und das Ventilelement drehbar innerhalb der Kammer untergebracht ist.

## Revendications

1. Canule d'irrigation et d'évacuation comprenant :
un boîtier (5) constituant une chambre ;
un élément de manchon généralement tubulaire (3) fixé au boîtier et en communication fluidique avec la chambre ;
un orifice d'instrument (11) dans le boîtier en communication fluidique avec la chambre, situé, généralement d'une manière opposée à et généralement alignée avec l'élément de_manchon ; et
des premier et second orifices de fluide (7, 9) dans le boîtier en communication fluidique avec la chambre, **caractérisée par**
un élément de vanne (13) apte à tourner dans le boîtier pour fermer sélectivement l'un ou les deux premier et second orifices de fluide tout en permettant continuellement qu'un instrument chirurgical soit inséré à travers l'orifice d'instrument, le boîtier et l'élément de vanne et l'élément de manchon.

2. Canule selon la revendication 1, où l'élément de vanne comprend un passage traversant en forme de Venturi pour permettre le passage de l'instrument chirurgical de l'orifice d'instrument à l'élément de manchon.

3. Canule selon la revendication 2, où ledit passage peut être amené en une communication fluidique avec les premier et second orifices pour réaliser un couplage de fluide sélectif entre ledit orifice de fluide et l'élément de manchon.

4. Canule selon l'une des revendications précédentes, comprenant en outre un élément formant commutateur (31) actionné par un déplacement de l'élément de vanne.

5. Canule selon l'une des revendications précédentes, où au moins un élément d'étanchéité de vanne (25) est disposé entre l'élément de vanne et le boîtier de manière à rendre étanche lesdits orifices de fluide à l'encontre d'une perte de fluide.

6. Canule selon l'une des revendications précédentes, où l'élément de vanne peut être retiré sélectivement du boîtier.

7. Canule selon l'une des revendications précédentes, où ledit orifice d'instrument comprend un élément d'étanchéité.

8. Canule selon la revendication 7, où l'élément d'étanchéité de l'orifice d'instrument comporte un joint à lèvre et un joint de diamètre externe.

9. Canule selon l'une des revendications précédentes, où les premier et second orifices sont dirigés dans le boîtier selon un angle aigu vers l'orifice d'instrument de manière à être approximativement en ligne avec l'élément de manchon.

10. Canule selon l'une des revendications précédentes, où l'élément de vanne et le boîtier ont des sections transversales circulaires correspondantes, et l'élément de vanne est reçu à rotation dans la chambre.
